# EUROPÄISCHE PATENTSCHRIFT

(11) **EP 2 520 317 B1**
(45) Veröffentlichungstag und Bekanntmachung des Hinweises auf die Patenterteilung: **09.07.2014**
(21) Anmeldenummer: 11075080.9
(22) Anmeldetag: 05.05.2011
(51) Int. Cl.: A61M 1/10

(54) **Blutpumpe**
Blood pump
Pompe à sang

(43) Veröffentlichungstag der Anmeldung: 07.11.2012
(73) Patentinhaber: Berlin Heart GmbH, 12247 Berlin (DE)
(72) Erfinder: Nüsser, Peter, 14532 Kleinmachnow (DE); Konstas, Georg, 97999 Igersheim (DE)
(74) Vertreter: Pfenning, Meinig & Partner GbR

(56) Entgegenhaltungen:
- WO-A1-01/70300
- WO-A1-98/41759
- US-A1- 2001 031 210

## Beschreibung

Die Erfindung bezieht sich auf das Gebiet der Blutpumpen.

Unter einer Blutpumpe wird hier und im Folgenden eine Pumpe verstanden, die der Unterstützung oder Herstellung eines Blutstroms innerhalb eines menschlichen oder tierischen Körpers dient und sich zur Implantation im Brustraum eines Menschen oder Tiers außerhalb des Herzens eignet. Bei linksventrikularen Blutpumpen (Left Ventricular Assist Devices, LVAD) besteht eine Verbindung zwischen der linken Herzhälfte und einem Einlass der Blutpumpe sowie zwischen einem Auslass der Blutpumpe und der vom Herzen abgehenden Aorta zur Unterstützung oder Herstellung des Blutkreislaufs durch den Körper (Körperkreislauf). Bei rechtsventrikularen Blutpumpen (RVAD) besteht eine Verbindung zwischen der rechten Herzhälfte und dem Lungenstamm, welcher zur linken und rechten Lungenarterie führt (oder eine direkte Verbindung zwischen dem RVAD und der linken und/oder rechten Lungenarterie), zur Unterstützung oder Herstellung des Blutkreislaufs durch die Lunge (Lungenkreislauf). Innerhalb der Blutpumpe wird das Blut durch einen Hohlkörper geleitet, welcher Teil eines Pumpengehäuses ist oder in einem solchen Pumpengehäuse angeordnet ist. In dem Hohlkörper ist ein rotierendes Laufrad mit einer Beschaufelung zur Erzeugung eines Drucks und einer daraus resultierenden Strömung des Bluts vorgesehen. So genannte Totalherzpumpen (Total Artificial Heart, TAH) beinhalten eine links- und eine rechtsventrikulare Blutpumpe zur Unterstützung oder Herstellung des gesamten Blutkreislaufs. Zur Herstellung der genannten Verbindungen zwischen der Blutpumpe und dem Herzen bzw. den Blutgefäßen werden Verbindungsschläuche und Rohre sowie gegebenenfalls Strömungskrümmer eingesetzt. Au-βerdem ist zur Energieversorgung und gegebenenfalls zur Steuerung der Blutpumpe wenigstens ein Kabelstrang erforderlich, der die Blutpumpe mit einem Energiespeicher und gegebenenfalls einer Steuerungseinheit verbindet. Alternativ kann die Pumpe transkutan mit Energie versorgt werden.

Ein Hauptproblem bei der Implantation und der Verwendung derartiger Blutpumpen ist der Raumbedarf solcher Blutpumpen und der Verbindungsschläuche sowie des Kabelstrangs im Brustraum in der Nähe des Herzens.

Eine weitere Schwierigkeit liegt in der Gefahr der Zerstörung von Blutkörperchen (Hämolyse) durch die Blutpumpe, insbesondere an mechanischen Lagern des Laufrads, Engführungen oder abrupten Richtungsänderungen der Blutströmung durch die Blutpumpe sowie durch starke Druckgradienten innerhalb der Blutpumpe. Bei der Konstruktion von Blutpumpen werden zu diesem Zweck häufig mechanische Lager des Laufrads durch eine magnetische und/oder hydrodynamische Lagerungsvorrichtung ersetzt.

Im Stand der Technik sind zahlreiche Blutpumpen bekannt. In der WO 2007/105842 A1 ist eine Blutpumpe offenbart, welche ein einen Rotor aufweisendes Laufrad umfasst, wobei das Laufrad hülsenartig auf einen ersten Leitkörper aufgesteckt ist. Unter einem Rotor wird hierbei und nachfolgend jeweils ein Motorrotor, d.h. eine Komponente des Motors verstanden, welcher in einigen Ausführungsbeispielen in einer Kavität des Laufrads vorgehalten wird und durch einen Magneten gebildet wird. Zudem umfasst die Pumpe ein Nachleitrad. Das Laufrad wird mit Hilfe magnetischer Lagerungen auf dem Leitkörper radial und zwischen den Leitkörpern axial gelagert.

In der US 6 015 272, US 6 244 835, US 6 447 265, US 2001/0031210 A1 und WO 01/70300 A1 wird jeweils eine zentrifugal wirkende Pumpe vorgeschlagen, die ein Pumpengehäuse mit einem zentralen Zapfen oder Dorn aufweist. Auf diesem Zapfen bewegt sich ein Laufrad mit einer zum Zapfen korrespondierenden Bohrung, wobei die äußere Form des Laufrads bzw. dessen Nabe kegelförmig ist. Die Beschaufelung des Laufrads ist auf dieser kegeligen äußeren Oberfläche angebracht und bewirkt die primär zentrifugal wirkende Pumpleistung. Zwischen der zylindrischen Innenoberfläche des Läufers und dem Zapfen bildet sich ein Blutspalt, in dem ein sekundärer Strömungspfad entsteht. Zur Lagerung des Laufrads werden in radialer Richtung Permanentmagnete und in axialer Richtung aktiv geregelte Magnete verwendet. Das Fluid wird durch einen spiralförmigen Auslass aus der Pumpe ausgestoßen.

Die US 5 370 509 und WO 98/41759 zeigen eine Blutpumpe, wobei der Leitkörper sich stromaufwärts kegelförmig verjüngt und auf dem kegelförmigen Leitkörper ein Laufrad mit einer sich in der radialen Ausdehnung verändernden Beschaufelung ausgebildet ist. Zum Antrieb des Laufrads befindet sich in der Nähe der stromabwärts gelegenen Wandung der Tangentialkammer ein innerhalb des Leitkörpers liegender Stator, welcher auf einen im Laufrad angeordneten Rotor wirkt. Aufgrund der Kegelform werden weitere Magnete zur axialen und radialen Lagerung eingesetzt, welche auch im Pumpengehäuse angeordnet sind.

Eine weitere Pumpe ist in der US 5 211 546 dargestellt. Es handelt sich hierbei um eine axial wirkende Pumpe, bei welcher das Laufrad hülsenartig auf einen dornartigen Leitkörper aufgesetzt wird. Weiterhin befinden sich stromaufwärts und stromabwärts des Laufrads Leiträder, welche über Leitschaufeln mit einer Wand des das Laufrad umgebenden Rohres verbunden sind. Die Leiträder weisen zur verbesserten axialen Lagerung des Laufrades Magnetlager auf.

Obgleich die vorab benannten Herzpumpen bisweilen bereits im praktischen Einsatz sind, besteht weiterhin der Bedarf an leistungsfähigen, platzsparenden Pumpen.

Aufgabe der vorliegenden Erfindung ist es, eine Blutpumpe vorzuschlagen, welche sich des vorgenannten Problems annimmt und eine Verbesserung bietet.

Diese Aufgabe wird erfindungsgemäß gelöst durch die Blutpumpe gemäß dem unabhängigen Patentanspruch 1. Weiterentwicklungen und weitere Ausführungsformen der Erfindung ergeben sich mit den abhängigen Ansprüchen und den nachstehend aufgeführten Ausführungsbeispielen.

Eine erfindungsgemäße Blutpumpe umfasst einen als Hohlkörper ausgebildeten axialen rohrförmigen Einlass, an welchen sich eine Tangentialkammer mit im Wesentlichen radialem Auslass anschließt. Des Weiteren ist ein sich in axialer Richtung erstreckender Leitkörper vorhanden, welcher entweder mit dem Einlass und/oder der Tangentialkammer verbundenen ist und auf diese Art im Wesentlichen koaxial zum axialen rohrförmigen Einlass im Strömungsverlauf gehalten wird. Auf den Leitkörper wird ein Laufrad mit einer dem Leitkörper zugewandten Innenfläche und einer dem Leitkörper abgewandten Außenfläche gelagert. Das Laufrad weist zudem eine auf seiner Außenfläche angeordnete Beschaufelung auf, sodass durch den rohrförmigen Einlass strömendes Blut axial in Richtung der Tangentialkammer transportiert wird und innerhalb der Tangentialkammer zum radialen Auslass gefördert wird.

Des Weiteren umfasst die Blutpumpe einen dem Leitkörper zugeordneten Stator und einen dem Laufrad zugeordneten Rotor, durch welche eine Rotation des auf dem Leitkörper angeordneten Laufrads bewirkt wird.

Erfindungsgemäß ragt zumindest ein Teilbereich des Leitkörpers von der Tangentialkammer in den Einlass. Da der Teilbereich auch in den rohrförmigen Einlass ragt, ist eine Verkleinerung der Tangentialkammer möglich und Teile bzw. Abschnitte des Laufrades und der auf dem Laufrad angeordneten Beschaufelung können in den axialen rohrförmigen Einlass verlagert werden. Auf diese Weise kann die Blutpumpe in ihrer axialen Länge verkürzt werden, da das Laufrad nicht länger vollständig innerhalb der Tangentialkammer bzw. der Spiralkammer gehalten werden muss.

Des Weiteren befindet sich zumindest ein Kraft auf den Rotor übertragender Abschnitt des Stators in diesem Teilbereich, so dass die Tangentialkammer weiterhin verkürzt ausgebildet werden kann. Da Teile des Stators nicht länger in der Tangentialkammer vorgehalten werden müssen, kann der vergleichsweise große Außenradius der Spiral- bzw. Tangentialkammer, der aus dem Stand der Technik bekannten Pumpen reduziert werden, da Teile der antreibenden Komponente des Motors in den Teilbereich des Leitkörpers verlagert werden. Aufgrund dieser Anordnung ist des Weiteren ein Austarieren des Laufrads auf dem Leitkörper deutlich vereinfacht. Da vorzugsweise ein Abschnitt des Laufrads derart auf dem Leitkörper angeordnet ist, dass der im Laufrad angeordnete Rotor durch den kraftübertragenden Abschnitt des Stators im Teilbereich des Leitkörpers angetrieben werden kann, und insbesondere für den Fall, dass der Rotor in einem axial betrachteten mittleren Abschnitt des Laufrades angeordnet ist, werden weniger Unwucht erzeugende Momente vom Stator auf den Rotor übertragen. Hierdurch vereinfacht sich insbesondere die radiale und axiale Lagerung des Laufrads auf dem Leitkörper wesentlich.

Da das Laufrad erfindungsgemäß in einer radialen Richtung durch ein hydrodynamisches Radiallager gelagert wird, wird im Zusammenspiel der Anordnung des Teilbereichs, des kraftübertragenden Abschnitts des Stators und des hydrodynamischen Radiallagers eine wesentliche konstruktive Vereinfachung gegenüber dem Stand der Technik ermöglicht. Aufgrund des Zusammenwirkens der vorgenannten drei Merkmale ist es insbesondere möglich, keine weiteren Antriebskomponenten radial außerhalb der Beschaufelung des Laufrads anzuordnen, d.h. insbesondere keine weiteren Antriebskomponenten auf einer Außenfläche des axialen rohrförmigen Einlasses bzw. auf einer Außenfläche der Tangentialkammer anzuordnen. Des Weiteren sind axial, d.h. stromabwärts der Tangentialkammer angeordnete Antriebs- oder Lagerungskomponenten nicht notwendig und verzichtbar. Auf diese Weise kann die Blutpumpe in einer besonders kleinen baulichen Form hergestellt werden, so dass die Implantation der Blutpumpe vereinfacht wird. Normalerweise wird der Pumpbetrieb derart eingestellt, dass der Druck am radialen Auslass größer als am axialen Einlass ist.

Des Weiteren ist erfindungsgemäß ein Verzicht auf ein stromaufwärts des Laufrads gelegenes Leitrad möglich. In diesem Falle ist der Leitkörper beispielsweise als von der Tangentialkammer in den Auslass ragender Dorn ausgebildet, auf welchen das Laufrad aufgesteckt wird. Hierdurch vereinfacht sich also auch das Zusammensetzen der verschiedenen Komponenten der Blutpumpe, wodurch Fehler beim Zusammenbau weiter vermieden werden und das Qualitätsmanagement der Blutpumpe vereinfacht wird.

In einer weiteren Ausführungsform ist das Laufrad in radialer Richtung ausschließlich durch ein vorzugsweise zwischen der Innenfläche des Laufrads und einer Leitkörperaußenfläche gebildetes hydrodynamisches Radiallager radial gelagert.

Durch die Ausbildung des Radiallagers als ausschließlich hydrodynamisches Lager wird die Anzahl der benötigten Komponenten zur Fertigung der Blutpumpe weiter reduziert. Zudem wird zusätzlicher Raum im Leitkörper freigegeben (da keine Radiallagerungsmagnete benötigt werden), welcher wiederum den kraftübertragenden Komponenten des Stators zur Verfügung gestellt werden kann. Hierdurch kann das durch den Stator erzeugbare Drehmoment aufgrund der größeren Dimensionierung der Statorelemente vergrößert werden.

In einer weiteren Ausführungsform wird das hydrodynamische Radiallager durch Ausnehmungen auf einer Leitkörperaußenfläche und/oder der Innenfläche des Laufrads gebildet. Durch entsprechende Kanäle der Innenfläche des Laufrads bzw. der Leitkörperaußenfläche kann das hydrodynamische Radiallager an das Leistungsspektrum der Blutpumpe angepasst werden. Die Spaltweiten zwischen der Leitkörperaußenfläche und der Innenfläche des Laufrads können beispielsweise zwischen 20 µm und 200 µm gewählt werden. Die Ausnehmungen bzw. Kanäle der Oberfläche erstrecken sich in axialer Richtung beispielweise von 1 mm bis 20 mm. Unter dem Leistungsspektrum der Blutpumpe sind hierbei die gewünschte Blutfördermenge, die dazu benötigte Umdrehungszahl (2000 U/min bis 35000 U/min) der Blutpumpe sowie der hierdurch zu erzeugende Blutdruck zu verstehen. Das Leistungsspektrum wird hierbei derart gewählt, dass die Pumpe sich zur Einstellung sehr unterschiedlicher Werte des Blutdrucks innerhalb eines Bereichs von etwa 5 mm Hg bis etwa 150 mm Hg bei einem stabilen konstanten, den physiologischen Bedingungen angepassten Volumenstrom zwischen 0 l/min bis 20 l/min eignet und sich auf diese Weise sowohl als RVAD als auch als LVAD einsetzen lässt bzw. sich zur Konstruktion einer etwaigen Totalherzpumpe eignet.

In einer weiteren Ausführungsform wird das hydrodynamische Radiallager derart ausgebildet, dass dieses zwei oder mehr Paare von Innenflächenabschnitten des Laufrads und jeweils dazu korrespondierenden Leitkörperaußenflächenabschnitten umfasst. Insbesondere bedeutet dies, dass beispielsweise derjenige Abschnitt des Leitkörpers, in welchem die kraftübertragenden Komponenten des Stators liegen, einen gegenüber den verbleibenden mit dem Laufrad überdeckten Leitkörperaußenflächenabschnitten größeren bzw. kleineren Durchmesser aufweist. Hierdurch ist eine weitere Verbesserung der hydrodynamischen Lagerung gegeben, wobei diese dem gewünschten Leistungsspektrum angepasst ist.

In einer weiteren Ausführungsform sind das Laufrad und der Leitkörper derart ausgebildet bzw. weisen zusätzliche Komponenten auf, welche in axialer Richtung, d.h. in Richtung der Achse des Leitkörpers und der koaxial hierzu verlaufenden Achse des Laufrads ein vorzugsweise passives magnetisches Axiallager zur axialen Lagerung vorgesehen ist. Um das Laufrad auf dem Leitkörper zu halten, kann entweder eine aktive magnetische Steuerung zur axialen Positionierung verwendet oder eine beispielsweise durch Permanentmagnete gebildete passive axiale Lagerung eingesetzt werden. Alternativ zur axialen Lagerung durch die Permanentmagnete, welche sowohl im Leitkörper als auch im Laufrad vorgehalten werden müssen, kann das axiale Magnetlager auch durch das Zusammenspiel von Stator und Rotor gebildet werden, da das Magnetfeld zwischen Rotor und Stator auch axiale Komponenten umfasst. Insbesondere wirkt das Magnetfeld zwischen dem Joch (beispielsweise das Blechpaket) des Stators und dem Permanentmagneten des Rotors auch axial zentrierend. Dabei wird die axiale Komponente des Magnetfelds des Motors so eingestellt, dass die axiale durch den Motor erzeugte Kraft der aufgrund der anströmenden Flüssigkeit erzeugten Kraft entgegenwirkt bzw. die axial zentrierende Komponente des Motormagnetfelds größer ist. Da in dieser Variante zusätzliche Permanentmagnete oder aktiv geregelte Magnete zur axialen Lagerung nicht notwendig sind, steht mehr Bauraum für den Motor zur Verfügung bzw. kann Bauraum eingespart und die Pumpe verkleinert werden.

Es ist bei der Verwendung eines axialen Lagers auf der Basis von Permanentmagneten insbesondere zur Ausnutzung des Bauraums innerhalb des Leitkörpers bzw. des Laufrades für die Motorkomponenten vorteilhaft, die etwaig notwendigen Komponenten des Axiallagers stromabwärts oder stromaufwärts des Rotors bzw. der die Kraft übertragenden Elemente des Stators anzuordnen. Insbesondere sollten die Komponenten zur axialen Lagerung vorzugsweise am stromaufwärts gelegenen Ende des Laufrads und dem dazu korrespondierenden Abschnitt des Leitkörpers sowie auf der stromabwärts gelegenen Seite des Laufrads und der dazu korrespondierenden stromabwärts gelegenen Seite des Leitkörpers angeordnet werden. Hierdurch lässt sich bereits mit relativ schwachen Magnetfeldern eine stabile axiale Lagerung erreichen. Entsprechend dem Stand der Technik sind die Magnete zur axialen Lagerung koaxial zur Achse des Leitkörpers oder der Leitkörperaußenfläche oder der Laufradinnenfläche magnetisiert.

Um eine vereinfachte Herstellung der Blutpumpe zu ermöglichen, kann der Leitkörper einen Hohlraum aufweisen, in welchem der Stator bzw. die kraftübertragenden Komponenten des Stators angeordnet sind. Weiterhin können weitere Hohlräume, beispielsweise für Komponenten der axialen Lagerung vorgesehen sein bzw. es kann ein einziger Hohlraum vorhanden sein, in welchem sämtliche Lager- und Statorkomponenten angeordnet sind. Hierdurch wird die Herstellung der Blutpumpe deutlich vereinfacht.

In einer weiteren Ausführungsform wird der Leitkörper mit einer stromabwärts gelegenen Wand der Tangentialkammer verbunden. Hierdurch ragt der Leitkörper als Dorn durch die Tangentialkammer hindurch in den rohrförmigen Einlass und es bedarf keiner weiteren Leiträder. Hieraus ergibt sich insbesondere der Vorteil, dass der Kabelstrang, welcher unter anderem zur Stromversorgung des Stators in den Leitkörper geführt werden muss, direkt durch die stromabwärts gelegene Wand der Tangentialkammer hindurch in den Leitkörper geführt wird. Insbesondere sind hier keine aufwendigen Konstruktionen zum Durchführen von Kabeln durch sich am Einlass oder an Seitenwänden der Tangentialkammer abstützende Leiträderschaufeln notwendig.

Vorzugsweise hat eine Kavität einer Tangentialkammer eine gegenüber einem Laufrad eine axiale Ausdehnung im Verhältnis 1:2 bis zu 1:7, vorzugsweise von 1:3 bis zu 1:4,5, d.h. das Laufrad ist länger als die axiale Ausdehnung der Kavität.

In einer weiteren Ausführungsform wird der Stator durch Wicklungen, Spulen oder ähnliches gebildet. Die Wicklungen können dabei geeignete, eine hohe Magnetfelddichte erzeugende Spulen sein. Geeignet sind sowohl verteilte Wicklungen wie auch Zahnspulen beliebiger Phasenzahl. Weitere geeignete Spulengeometrien ergeben sich für den Fachmann aus dem Stand der Technik.

Um die Magnetfeldstärke zusätzlich zu erhöhen und somit einen verbesserten Kraftübertrag zu gewährleisten, kann vorgesehen sein, einzelne Wicklungen oder sämtliche Wicklungen um vorzugsweise einen oder mehrere Eisenkerne auszuführen, um das in den Eisenkernen induzierte Magnetfeld zusätzlich zum Antrieb des Rotors auszunutzen. Dementsprechende Anordnungen zum Aufbau eines Stators sind im Stand der Technik hinlänglich bekannt.

In einer weiteren Ausführungsform ist das Laufrad zumindest im Teilabschnitt des Leitkörpers, welcher in den axialen rohrförmigen Einlass ragt, zylindermantelförmig ausgebildet. Vorzugsweise ist das Laufrad nicht nur in diesem Teilabschnitt sondern über die gesamte Länge hinweg zylindermantelförmig ausgebildet. Insbesondere ist unter einem Zylindermantel hierbei im Wesentlichen ein gerades Rohr zu verstehen, dessen Symmetrieachse koaxial zur Achse des Leitkörpers ausgebildet ist.

Vorzugsweise umfasst das Laufrad einen Hohlraum, wobei der Rotor bzw. die den Rotor bildenden Elemente in diesem Hohlraum angeordnet sind. Alternativ hierzu können die den Rotor bildenden Komponenten mit einem das Laufrad bildenden Material vergossen werden.

Der Rotor wird vorzugsweise durch mindestens einen alternierend magnetisierten Permanentmagnetring (vorzugsweise im Wesentlichen kreisförmig)gebildet, kann jedoch weitere derartige Permanentmagnetringe umfassen. Auch weitere Komponenten zur Bildung des Rotors können vorgesehen werden. Unter der alternierenden Magnetisierung sind hierbei radial oder diametral zur Achse des Leitkörpers im Teilabschnitt oder radial oder diametral von der Achse des Teilabschnitts weg gerichtete Magnetisierungen zu verstehen. So sind beispielsweise in einem Zweipolmotor ein Permanentmagnetsegment mit einer radial oder diametral von der Achse weg gerichteten Magnetisierung und ein Permanentmagnetsegment mit einer zur Achse gerichteten Magnetisierung vorgesehen. Um den Kraftübertrag zu erhöhen, können axial betrachtet zahlreiche derartige Permanentmagnetringe angeordnet werden, bevorzugt ist jedoch ein einzelner Permanentmagnetring mit einer sich über die axiale Ausdehnung des Stators erstreckenden axialen Ausdehnung.

In einer Ausführungsform wird ein Permanentmagnetring durch einen einstückigen Ringmagneten gebildet oder aus einer Vielzahl an jeweils einzeln magnetisierten Teilsegmenten zusammengesetzt. Unter einem Ringmagnet ist hierbei ein einstückiger Ring aus einem magnetischen Material zu verstehen, welcher zuerst als Ring geformt wird und welcher anschließend magnetisiert wird, d.h. es werden unterschiedliche Abschnitte des Rings nachträglich magnetisiert.

Wird der Permanentmagnetring aus einer Vielzahl von Segmenten, d.h. mehr als zwei Segmenten, zusammengesetzt, so kann jedes einzelne Segment zunächst magnetisiert und die Segmente anschließend zu einem Ring zusammengesetzt werden. Dabei sind benachbarte Segmente jeweils alternierend magnetisiert. Entweder können die einzelnen Segmente dabei aneinander anliegen oder benachbarte Segmente können durch Abstandshalter von einander beabstandet werden. Abstandshalter können beispielsweise aus paramagnetischen Materialen gefertigt werden.

In einer weiteren Ausführungsform ist das Laufrad derart auf dem Leitkörper angeordnet, dass die Beschaufelung des Laufrads sowohl innerhalb der Tangentialkammer als auch im Einlass liegt. Hierdurch ist es möglich, die axiale Förderung des Blutes durch den rohrförmigen Einlass zu gewährleisten, und somit eine besonders blutschonende Förderung des Blutes in die Radialkammer bzw. Tangentialkammer bzw. Spiralkammer zu gewährleisten. Auch eine schneckenförmige Kammer (Schneckenkammer) ist möglich. Während bei einer Spiralkammer die Kammer sich von einer Zunge zum Auslass hin radial aufweitet, d.h. das Volumen der Kammer pro Winkelsegment nimmt aufgrund der radialen Aufweitung zu, nimmt bei der Schneckenkammer das Volumen der Kammer pro Winkelsegment auch in axialer Richtung zu. Selbstverständlich kann auch eine Kammer mit radialem Auslass gewählt werden, deren Volumen pro Winkelsegment (von einer Zunge gesehen) sowohl in radialer Richtung als auch in axialer Richtung zunimmt und so eine Kombination aus Spiral- und Schneckenkammer bildet. Unter den Begriff der Tangentialkammer werden in dieser Anmeldung sowohl Spiral- als auch Schneckenkammern als auch deren Kombinationen zusammengefasst.

Erst nachdem das Blut einen axialen Vorschub erhalten hat, gelangt es in die Tangentialkammer, aus welcher es lediglich einen einzigen radialen Auslass gibt (gegebenenfalls sind auch mehrere Auslässe denkbar), so dass die blutschonende axiale Förderung mit dem platzsparenden radialen Auslass kombinierbar wird.

Die Tangentialkammer weist vorzugsweise mindestens eine Zunge auf, kann jedoch auch mehrere Zungen umfassen. Insbesondere kann die Anzahl der Zungen mit der Anzahl der Schaufeln des Laufrads korrespondieren, um eine besonders effiziente und schonende Förderung der Flüssigkeit zu gewährleisten. Eine derartige Tangentialkammer wird beispielsweise als Doppel-oder Mehrfachspiralkammer bezeichnet.

In einer weiteren Ausführungsform ist die Beschaufelung als eine auf der Außenfläche angeordnete Wendel ausgebildet. Zur Erzielung des axialen Vortriebs des Bluts ist die wendelförmige Beschaufelung ein oder mehrgängig ausgebildet. Die Wendel kann also eine oder mehrere, vorzugsweise zwei bis sechs wendelförmige Schaufeln aufweisen, welche das Laufrad bezüglich der Rotationsachse jeweils teilweise vollständig oder mehrfach umlaufen. Die Schaufeln schlie-βen mit der Achse des Laufrads einen nicht verschwindenden Anstellwinkel ein, über den die Förderwirkung des Laufrads einstellbar ist.

Bei einer Rotation des Laufrads um die Achse überträgt die Wendel eine axial ausgerichtete Kraft auf an die Schaufeln angrenzende Volumenanteile des Blutes. Auf diese Weise wird das Blut mittels der Wendel durch den rohrförmigen Einlass hindurch "geschoben". Die Übertragung des axialen Vortriebs auf das Blut mittels der Wendel hat sich hierbei als besonders blutschonend erwiesen. Im Gegensatz hierzu sind die Rotorschaufeln von Radialpumpen grundsätzlich parallel zur Rotationsachse des Laufrads ausgerichtet bzw. weisen lediglich einen kleinen Anstellwinkel auf. Die Rotorschaufeln der Radialpumpen übertragen überwiegend eine tangential ausgerichtete Kraft, auf die an die Rotorschaufeln angrenzenden Volumenanteile des Blutes jedoch geringere axiale Kräfte. Die an die Rotorschaufeln angrenzenden Volumenanteile werden somit bei Radialpumpen in einer Rotationsbewegung um die Drehachse des Laufrades versetzt, so dass das Blut über Zentrifugalkräfte radial nach außen beschleunigt und aus der Pumpe durch den Auslass heraus befördert wird. Einen axialen Vortrieb erhält das Blut bei Radialpumpen, welche auch als Zentrifugalpumpen bezeichnet werden, ausschließlich durch eine axiale Sogwirkung, welche durch das Ausströmen des Bluts aus der Pumpe hervorgerufen wird. Dahingegen wird bei der erfindungsgemäßen Pumpe das Blut innerhalb der Pumpe durch die Wendel zumindest teilweise in axialer Richtung "geschoben".

Die Wendel ist vorzugsweise durchgängig ausgestaltet. Vorzugsweise erstreckt sich die Wendel über mindestens 80% der axialen Ausdehnung (Länge) des Laufrads, besonders bevorzugt über mindestens 90% der axialen Ausdehnung des Laufrads. Es kann aber auch vorgesehen sein, dass sich die Wendel über die gesamte Länge des Laufrades erstreckt. Auf diese Weise eignet sich das Laufrad zur Erzeugung eines besonders schonenden und verwirbelungsarmen axialen Vortriebs des Bluts. Zudem lässt sich die Förderwirkung der Wendel durch eine Änderung des Anstellwinkels der Wendel, bzw. einer lokalen Steigung der Wendel K besonders leicht modifizieren. Die lokale Steigung K der Wendel, gemessen an einer vorgegebenen axialen Höhe der Wendel, ist dabei wie üblich definiert als das Verhältnis einer axialen Verschiebung eines an die Wendel angrenzenden infinitesimal kleinen Volumenelements ΔX an der genannten Höhe der Wendel, die durch eine Drehung des Laufrads um einen infinitesimal kleinen Drehwinkel Δϕ hervorgerufen wird, geteilt durch den Drehwinkel Δϕ, also K=ΔX/Δϕ. Eine lokale Ganghöhe H der Wendel ergibt sich damit als axiale Verschiebung eines an die Wendel angrenzenden Volumenelements durch die Wendel, gemessen an einer vorgegebenen axialen Höhe der Wendel, die durch eine ganze Drehung des Laufrads hervorgerufen wird, also H=2πK.

Vorzugsweise liegt die lokale Steigung der Wendel über die gesamte axiale Erstreckung der Wendel hinweise in einem Bereich zwischen etwa 0,5 mm und 20 mm. Dies entspricht einer lokalen Ganghöhe zwischen etwa 3 mm und 130 mm. In einer Weiterentwicklung ist vorgesehen, dass die lokale Steigung der Wendel an einer Zuströmseite des Laufrads, d.h. stromaufwärts gelegen, zwischen 0,5 mm und 2 mm liegt. Dies entspricht einer lokalen Ganghöhe zwischen etwa 3 und 13 mm. An einer Abströmseite des Laufrads beträgt die lokale Steigung zwischen 3 mm und 20 mm. Dies entspricht einer lokalen Ganghöhe zwischen etwa 18 und 130 mm. Es kann außerdem vorgesehen sein, dass die lokale Steigung und die lokale Ganghöhe der Wendel zur Abströmseite des Laufrads hin stetig zunehmen.

Als mittlere Steigung der Wendel ist die Mittelung der lokalen Steigung über die gesamte axiale Erstreckung der Wendel definiert. Analog hierzu ist als mittlere Ganghöhe der Wendel die Mittelung der lokalen Ganghöhe über die gesamte axiale Erstreckung der Wendel definiert. In einer bevorzugten Ausführungsform liegt die mittlere Steigung zwischen etwa 1 mm und 5 mm, das entspricht etwa einer mittleren Ganghöhe von etwa 6 mm bzw. etwa 30 mm.

Prinzipiell führt eine größere Steigung, bzw. ein kleinerer Anstellwinkel, der Wendel zu einer stärkeren Übertragung von Drall auf das Blut. Zusätzlich zum axialen "Vorwärtsschieben" des Blutes durch die Wendel wird erfindungsgemäß auch die Beaufschlagung des Blutes mit Drall (Rotationsenergie) zum Druckaufbau eingesetzt. In den Ausführungsformen ist daher vorgesehen, dass die Wendel bis in die Tangentialkammer der Blutpumpe hineinreicht. Da aus dieser Tangentialkammer das Blut über den Auslass tangential ausströmt, wird somit neben der axialen Strömungsenergie des Blutes auch die Rotationsenergie des Blutes zum Druckaufbau effizient ausgenutzt.

Vorzugsweise ist eine Außenkontur der Wendel im Wesentlichen zylinderförmig ausgestaltet, d.h. die radiale maximale Ausdehnung der Wendel bleibt über dieser Länge konstant. Ebenso kann auch die Mantelfläche des Laufrads, welche die Wendel trägt, im Wesentlichen zylindrisch ausgestaltet sein. Ferner ist das Laufrad in axialer Richtung vorzugsweise langgestreckt und weist einen maximalen Durchmesser auf, der vorzugsweise nicht größer ist als 50% der axialen Erstreckung der Beschaufelung des Laufrads, besonders bevorzugt nicht größer als 30% der axialen Erstreckung des Laufrads. Durch eine langgestreckte Form des Laufrads kann eine besonders schlanke Bauform der Blutpumpe erzielt werden. Ferner lässt sich auf diese Weise eine besonders lange axiale Erstreckung der wendelförmigen Beschaufelung des Laufrads erzielen für eine besonders gleichmäßige und blutschonende axiale Beförderung des Blutes.

In einer Weiterentwicklung ist vorgesehen, dass eine maximale radiale Ausdehnung der Beschaufelung, das heißt die maximale Höhe der Beschaufelung, kleiner ist als 50% eines maximalen Gesamtradius' des Laufrads (gemessen senkrecht zur Rotationsachse und einschließlich der Beschaufelung), vorzugsweise kleiner als 30% des maximalen Gesamtradius des Laufrads. Typischerweise liegt die maximale Höhe der Beschaufelung in einem Bereich zwischen 1 mm und 4 mm, besonders bevorzugt in einem Bereich zwischen 2 mm und 3 mm.

Es kann auch vorgesehen sein, dass die Beschaufelung durch mindestens eine Schaufel gegeben ist, wobei die mindestens eine Schaufel eine maximale Dicke aufweist (gemessen senkrecht zur Rotationsachse und senkrecht zur Höhe der Beschaufelung), die geringer ist als 10% eines maximalen Gesamtumfangs des Laufrads (gemessen senkrecht zur Rotationsachse und einschließlich der Beschaufelung), vorzugsweise beträgt die maximale Dicke der Schaufel weniger als 5% des maximalen Gesamtumfangs. Typischerweise liegt die maximale Dicke in einem Bereich zwischen 0,5 mm und 3 mm, besonders bevorzugt in einem Bereich zwischen 1 mm und 2 mm.

In einer weiteren Ausführungsform ist vorgesehen, dass die Innenfläche des Laufrads eine zylinderförmige Innenfläche ist und/oder die Außenfläche des Laufrads ebenfalls eine zylinderförmige Fläche ist, auf welcher die Beschaufelung angeordnet ist.

Hierbei ist vorgesehen, dass das Laufrad einen maximalen Durchmesser aufweist, welcher nicht größer ist als 50% einer axialen Erstreckung der Beschaufelung. Hierdurch wird eine schonende Energieübertragung auf das Blut realisiert.

In einer weiteren Ausführungsform ist die maximale radiale Ausdehnung der Beschaufelung kleiner als 50% eines maximalen Gesamtradius des Laufrads inklusive der Beschaufelung. Hierbei wird die Beschaufelung von der Außenfläche des Laufrads bis an seinen radialen Rand hin bemessen.

In einer weiteren Ausführungsform ist die Beschaufelung durch mindestens eine Schaufel gegeben, wobei die mindestens ein Schaufel eine maximale Dicke aufweist, die geringer ist als 10% eines maximalen Gesamtumfangs des Laufrads. Hierbei kann die Schaufel auch einen Wendelabschnitt bezeichnen.

Hierdurch werden gute Förderverhältnisse für Blut gesichert und Reibungsverluste in der Pumpe minimiert.

Vorzugsweise beträgt der Radius des Laufrads inkl. Beschaufelung weniger als 100% des Innendurchmessers des rohrförmigen Einlasses, jedoch mehr als 90% des Innendurchmessers, um so Rückströme an Blut durch einen zwischen dem Innendurchmesser des Einlasses und dem Außendurchmesser der Beschaufelung gebildeten Rückstromkanal zu verhindern. Vorzugsweise trägt dieser Rückstromkanal nicht wesentlich bzw. nicht nennenswert zur hydrodynamischen Lagerung bei, kann jedoch ggf. als Dämpfungsglied verwendet werden. Weitere Kombinationen von Blutpumpen ergeben sich aus den weiteren Aspekten der Erfindung. Insbesondere ist eine Variante vorgesehen, bei welcher die Blutpumpe zwar nicht zwangsläufig ein hydrodynamisches Radiallager aufweist, jedoch weiterhin zumindest ein Teilbereich des Leitkörpers in den Einlass ragt und zumindest ein Kraft auf den Rotor übertragender Abschnitt des Stators in diesem Teilbereich angeordnet ist. Dadurch, dass das Laufrad an seinem äußeren radialen Durchmesser auch durch den Spalt zwischen dem Innendurchmesser des Einlasses und dem Außendurchmesser des Laufrads gelagert werden kann, sind Bauformen ohne rein hydrodynamisches Radiallager von der Erfindung umfasst. Insbesondere ist es eine weitere Variante der Erfindung, dass eine Pumpe ohne radiales hydrodynamisches Lager gebildet wird bzw. weitere Lager aufweisen kann, jedoch ein Teilbereich des Leitkörpers weiterhin in den Einlass ragt und der Leitkörper im Teilbereich eine zylinderförmige Leitkörperaußenfläche umfasst, auf welcher das Laufrad zumindest im Teilabschnitt zylindermantelförmig ausgebildet ist. Bereits durch diese Ausführungsformen können zahlreiche Vorteile der Erfindung realisiert werden.

Allgemein sei erwähnt, dass mit einer Pumpe gemäß dem unabhängigen Anspruch eine kleinere Dimensionierung gegenüber handelsüblichen Blutpumpen möglich ist. Da der Stator innenliegend im Leitkörper angeordnet ist und das Laufrad den Rotor umfassend auf dem Leitkörper gelagert ist, müssen sowohl der axiale rohrförmige Einlass als auch die Tangential- bzw. Spiralkammer keine weiteren Komponenten des Motors oder das Lager aufnehmen. Hierdurch wird es möglich, die Wandungen des Einlasses sowie der Tangentialkammer gegenüber herkömmlichen Modellen besonders dünn auszubilden.

Weitere Ausführungsformen werden dem Fachmann durch die nachfolgenden Ausführungsbeispiele nahegelegt. Es wird darauf hingewiesen, dass die Merkmale der verschiedenen Ausführungsformen miteinander kombinierbar sind und nicht sämtliche Merkmale eines Ausführungsbeispiels notwendig sind, um das jeweilige Ausführungsbeispiel mit einer anderen Ausführungsform zu kombinieren. Es zeigen
- Fig. 1: einen Längsschnitt durch eine erfindungsgemäße Variante einer Pumpe;
- Fig. 2: eine perspektivische Ansicht der Pumpe der Fig. 1;
- Fign. 3a und 3b: Varianten einer Spiralkammer der Pumpe der Fig. 1;
- Fig. 4: ein Längsschnitt mit Fokus auf eine Variante des Leitkörpers;
- Fign. 5a und 5b: Längs- und Querschnitt durch eine Variante des Laufrads für eine erfindungsgemäße Pumpe;
- Fign. 6a und 6b: einen Längsschnitt durch eine weitere erfindungsgemäße Variante einer Pumpe in einer Variante eines ausschließlich hydrodynamischen Radiallagers und einer Axiallagerung durch den Motor;
- Fign. 7a bis 7c: eine Variante einer Schneckenkammer;
- Fig. 8: eine Variante einer Doppelspiralkammer.

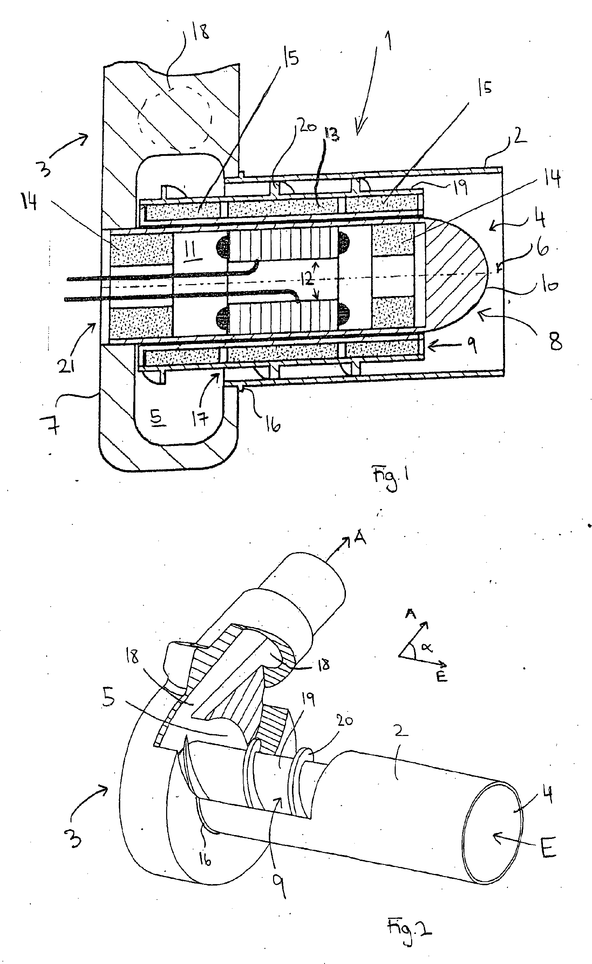

In der Fig. 1 ist eine Pumpe 1 im Längsschnitt dargestellt. Die Pumpe umfasst ein Rohr 2, welches den axialen rohrförmigen Einlass der Blutpumpe bildet. Stromabwärts ist das Rohr 2 mit der Spiralkammer 3 verbunden. Der Hohlraum 4 des Rohres 2 erstreckt sich dabei in die Kavität 5 der Spiralkammer, welche in einem in der Fig. 1 nicht näher dargestellten Auslass 18 mündet. Dabei schließen die Achse 6 des Rohres 2 und eine Achse des Auslasses einen Winkel von 90° ein. Prinzipiell sind jedoch auch Winkel zwischen 45° und 135° möglich. An der rückseitigen Wand 7 der Spiralkammer ist ein Leitkörper 8 angeordnet, welcher sich durch die Kavität 5 hindurch in den Hohlraum 4 des Rohres 2 erstreckt.

Der Leitkörper 8 erstreckt sich zapfenartig in den Hohlraum 4, wobei der Leitkörper 8 bis auf die nicht durch das Laufrad 9 überdeckte Kappe 10 im Wesentlichen die Form eines Zylindermantels aufweist, in welchem ein Hohlraum 11 angeordnet ist. In dem Hohlraum 11 befindet sich der Stator 12 des Motors der Blutpumpe, welcher mit dem Rotor 13 des Laufrads 9 in Wirkverbindung steht und eine Drehung des Laufrads 9 bewirkt. Im Hohlraum 11 sind Permanentmagnetsysteme 14 angeordnet, welche eine Magnetisierung parallel zur Achse 6 aufweisen. Die Permanentmagnetsysteme 14 bilden zusammen mit den im Laufrad 9 angeordneten Permanentmagnetsystemen 15, ein passives axiales Lager, welches einen axialen Versatz des Laufrads 9 auf dem Leitkörper 8 verhindert.

Um eine passgenaue Verbindung zwischen dem Rohr 2 und der Spiralkammer 3 herzustellen, weist das Rohr 2 einen Kragen 16 auf, wobei der Durchmesser des Kragens 16 größer ist als der Durchmesser der Öffnung 17 der Spiralkammer, so dass das Rohr nicht beliebig tief in die Spiralkammer geschoben werden kann, sondern der Kragen 16 auf der Außenseite der Spiralkammer 3 einen Anschlag bildet. Eine dauerhafte Verbindung zwischen dem Rohr 2 und der Spiralkammer 3 kann beispielsweise über ein Gewinde, durch Verschweißen oder Verkleben der beiden vorgenannten Elemente erfolgen. Obgleich in dem hier dargestellten Ausführungsbeispiel das Rohr 2 und die Spiralkammer 3 als zwei voneinander getrennte Teile vorgefertigt werden, welche erst anschließend verbunden werden, ist es ebenso möglich, einen Einlass stoffschlüssig an die Spiralkammer 3 anzuschließen, so dass die Funktion des Rohres 2 durch den stoffschlüssig mit der Spiralkammer 3 verbundenen Einlass bewirkt wird.

Auf weitere Einzelheiten soll in den nachfolgenden Figuren näher eingegangen werden.

In der Fig. 2 ist eine perspektivische Aufsicht auf die Pumpe der Fig. 1 dargestellt. Das Rohr 2 ist deutlich erkennbar, ebenso der Kragen 16, mit welchem das stromabwärts gelegene Ende des Rohres 2 auf einer stromaufwärts gelegenen Wand der Spiralkammer 3 abgestützt wird. Im vorliegenden Beispiel ist der Außendurchmesser des Rohres 2 derart gewählt, dass dieser passgenau in den Innendurchmesser der Öffnung 17 der Spiralkammer 3 passt.

In der Fig. 2 ist ein Teilbereich der Wandung, sowohl des Rohres als auch der Spiralkammer 3 zur übersichtlichen Darstellung ausgespart, so dass das sich durch das Rohr 2 in die Kavität 5 der Spiralkammer 3 erstreckende Laufrad 9 sowie die Form der Kavität 5 und dessen Auslass 18 verdeutlicht werden. Auf dem Laufrad 9 ist neben der Laufradaußenfläche 19 eine Beschaufelung 20 zu erkennen, welche im vorliegenden Ausführungsbeispiel als durchgehende Wendel ausgebildet ist. Hinsichtlich der möglichen Ausführungsform der Wendel wird auf den allgemeinen Beschreibungsteil verwiesen.

Mit Hilfe der Beschaufelung 20 des Laufrads 9 wird beim Betrieb des Motors Blut in Richtung E vom Rohr 2 in die Kavität 5 gepumpt und anschließend auch aufgrund der Umfangskomponente der Fluidgeschwindigkeit durch den Auslass 18 in der Richtung A ausgestoßen. Der Winkel α zwischen den Richtungen E und A beträgt hierbei α=90°, kann jedoch im Intervall α =45°-135°variiert werden. In den Fign. 1 und 2 ist deutlich erkennbar, dass keinerlei elektrische Antriebs-oder Lagerungskomponenten an den Außenwänden des Rohres bzw. der Spiralkammer angeordnet sind, mit Ausnahme der rückseitigen Wand 7 der Spiralkammer (jedoch indirekt über den Leitkörper 8). Sämtliche Komponenten des Stators und des Axiallagers sind im Hohlraum 11 des Leitkörpers 8 enthalten und die Spiralkammer 3 selbst weist keinerlei elektrische Komponenten auf. Dies trägt wesentlich zur Vereinfachung des Aufbaus bei.

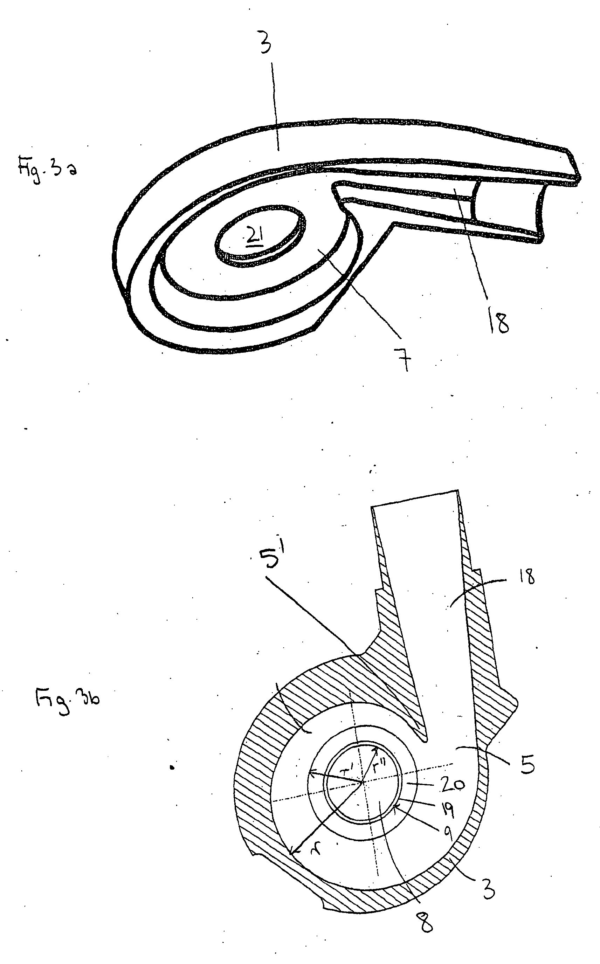

Zur deutlicheren Darstellung der Spiralkammer 3 wird in der Fig. 3a ein Querschnitt durch die Spiralkammer 3 dargestellt. Die rückseitige Wand 7 umfasst eine Öffnung 21, welche einen Durchmesser aufweist, welcher größer ist als der Außendurchmesser eines einzuführenden Leitkörpers. Vorzugsweise wird der Radius der Öffnung 21 derart gewählt, dass die Innenseite der die Öffnung begrenzenden Wände passgenau mit dem Außendurchmesser eines etwaigen Leitkörpers abschlie-βen. Der radiale Auslass 18 ist ebenfalls deutlich erkennbar. Vorzugsweise wird die Spiralkammer aus einem biokompatiblen Material, wie beispielsweise Titan, Keramik oder beschichteten Kunststoffen, hergestellt.

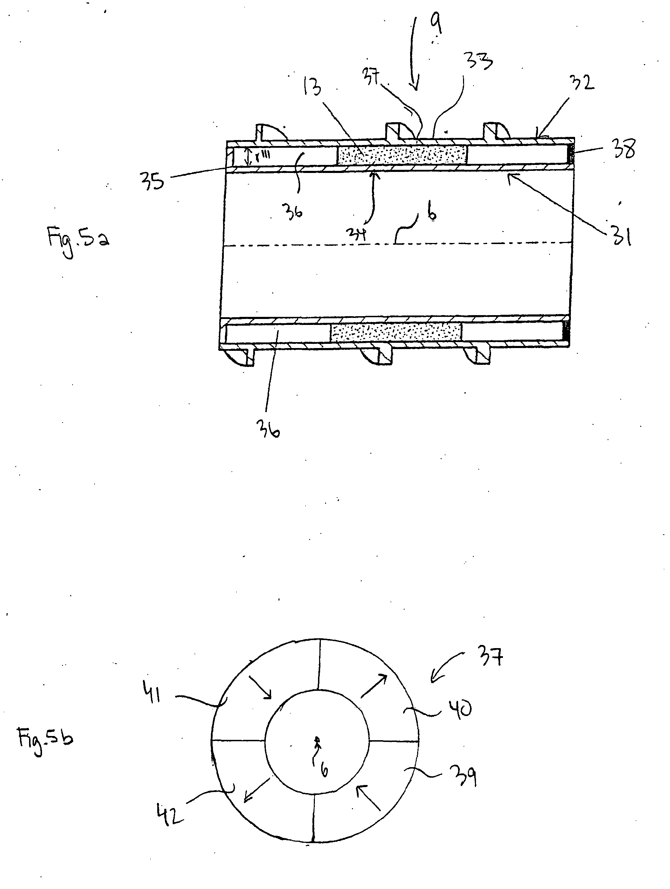

In der Fig. 3b ist eine von der Fig. 3a verschiedene Perspektive der Rückwand der Spiralkammer 3 dargestellt. Hierbei ist eine Ansicht vom Rohr 2 auf die Spiralkammer 3 dargestellt. Es ist deutlich erkennbar, dass der Radius r der Kavität 5 der Spiralkammer 3 vom der Zunge 5' gegen den Uhrzeigersinn hin zunimmt. Die Zunahme erfolgt bis zum Eingang in den Auslass 18. Neben der Spiralkammer 3 ist auch der Leitkörper 8 dargestellt, welcher einen Innenradius r" besitzt. Des Weiteren ist das Laufrad 9 erkennbar, insbesondere der den Rotor aufnehmende Hohlraum 36 (siehe Fig. 5a) und die Beschaufelung 19, welche einen gleichbleibenden Durchmesser von r' aufweist. Der Radius r' ist derart gewählt, dass zwischen einem Innenradius des Rohres 2 und dem Außenradius r' der Beschaufelung 19 ein Spalt verbleibt. Hierdurch wird eine besonders schonende Förderungsweise des Blutes ermöglicht.

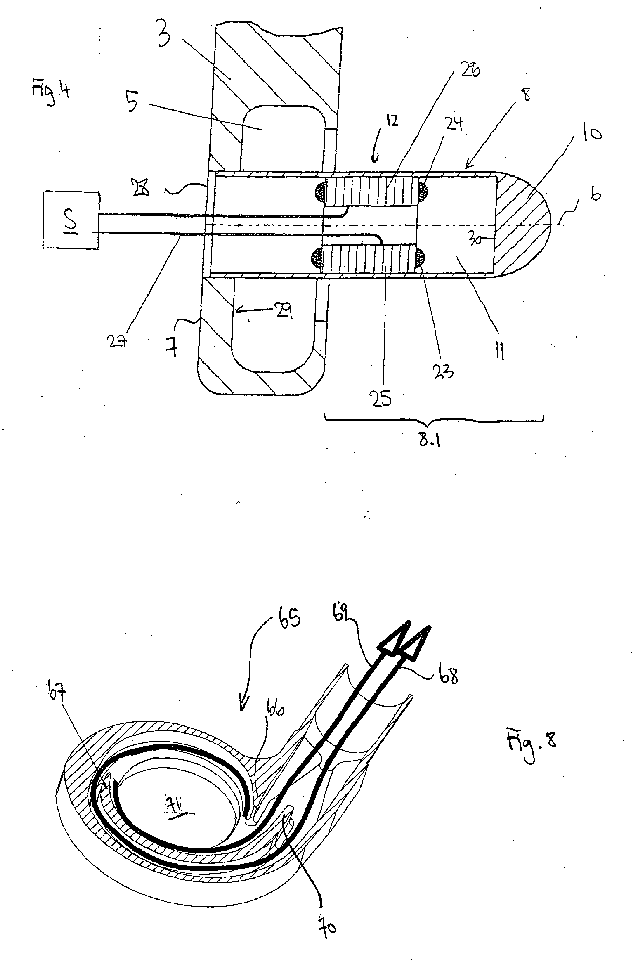

In der Fig. 4 ist sowohl die Spiralkammer 3 als auch der in die rückseitige Wand 7 der Spiralkammer 3 eingesetzte Leitkörper 8 dargestellt. Es ist deutlich zu erkennen, dass ein Großteil des Leitkörpers 8, insbesondere der Abschnitt 8.1, sich aus der Spiralkammer heraus erstreckt und, wie in der Fig. 1 zu sehen, in das Rohr 2 ragt. Die Spitze 10 des Leitkörpers 8 ist gerundet ausgeführt, um eine möglichst schonende Wirkung bei der Blutförderung zu bewirken.

Im Hohlraum 11 befindet sich der Stator 12, welcher Wicklungen 23 und 24 umfasst, welche um jeweils einen Metallkern 25 bzw. 26 angeordnet sind. Die Wicklungen sind aus Kupferdraht gewickelt und sind mit der Verkabelung 27 verbunden und können so eine Bewegung des auf dem Leitkörper 8 angeordneten Laufrads 9 bewirken. Hierzu ist eine nicht näher erläuterte Steuereinheit S vorgesehen, welche die entsprechende und aus dem Stand der Technik bekannte Umpolung des Magnetfelds, welches durch die Wicklungen 23 und 24 erzeugt wird, bewirkt.

Die Kavität 11 ist zum einen durch die Spitze 10 und zum anderen durch den Deckel 28 verschlossen. Aus dem Deckel heraus ragt lediglich die Verkabelung 27, alle weiteren Statorkomponenten befinden sich ausschließlich im Hohlraum 11.

Des Weiteren ist erkennbar, dass die Eisenkerne 25, 26 des Stators 12 derart in dem Leitkörper 8 bzw. in dem Hohlraum 11 des Leitkörpers 8 angeordnet sind, dass mehr als 90% des Volumens der die Kraft übertragenden Teile des Stators, d.h. die Eisenkerne bzw. die Wicklungen nicht in der Kavität 5 der Spiralkammer 3 liegen, sondern, wie in der Fig. 1 erkennbar, in das Rohr 2 hineinragen.

Zugleich ist erkennbar, dass der Stator - abgesehen von der Verkabelung 27 - ungefähr im mittleren Drittel des Leitkörpers 8 (betrachtet in axialer Richtung, d.h. in Richtung der Achse 6) angeordnet ist. Insbesondere ist der Stator 12 bzw. sind die die Kraft übertragenden Komponenten des Stators 12 derart im Hohlraum 11 angeordnet, dass von der Innenseite 29 der rückseitigen Wand 7 bis zum stromaufwärts gelegenen Ende 30 des Hohlraums 11 der Stator im Wesentlichen auch im mittleren Drittel des Hohlraums angeordnet ist. Grund hierfür ist eine im Wesentlichen symmetrische Anordnung der Antriebskomponenten. Allgemein bewirkt eine symmetrische Anordnung eine symmetrische Radialkraftverteilung.

So bewirkt ein im mittleren Drittel angeordneter Stator eine bezogen auf die Länge des Laufrades symmetrische Verteilung der radialen Kräfte zwischen Rotor und Stator des Motors.

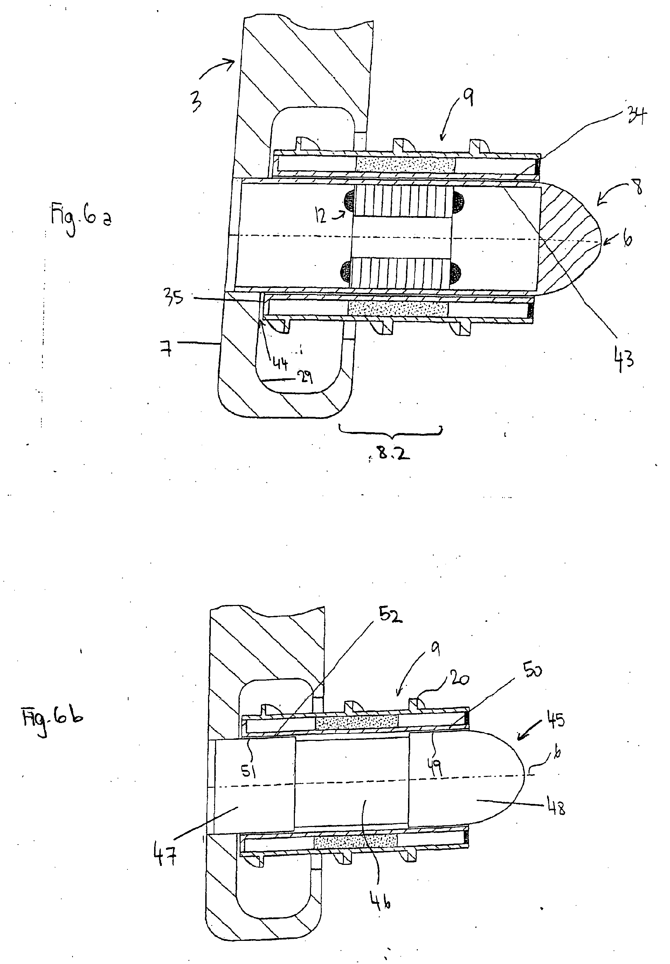

Obgleich in der Variante der Pumpe der Fig. 1 Permanentmagnete zur axialen Lagerung eingezeichnet sind, bedarf es dieser Permanentmagnete nicht, falls die axiale Lagerung durch den Stator und den Rotor vermittelt wird. Somit ist das in der Fig. 4 jeweils dargestellte Ausführungsbeispiel (siehe auch Fign. 5a und 6) auch ohne Permanentmagnetsysteme 14 bzw. 15 ausführbar.

Im Gegensatz zu der in der Fig. 4 dargestellten Variante ist es selbstverständlich auch möglich, ein Axiallager im mittleren Drittel anzuordnen und den Stator entweder im vorderen oder hinteren Drittel des Leitkörpers oder sowohl im vorderen als auch im hinteren Drittel des Leitkörpers 8 anzuordnen. Hierdurch werden jedoch die Verkabelung und die Ansteuerung der Wicklungen aufwendiger.

Nachfolgend wird in den Fign. 5a und 5b das Laufrad näher beschrieben. Das Laufrad 9 ist aus einem innen liegenden Zylinder 31 und einem außen liegenden Zylinder 32 aufgebaut, wobei die Außenseite des außen liegenden Zylinders 32 die Außenfläche 33 bildet, und die Innenseite des innen liegenden Zylinders 31 die Innenfläche 34 bildet. Sowohl der innen liegende Zylinder 31 als auch der außen liegende Zylinder 32 haben Zylinderachsen, welche koaxial zur eingezeichneten Achse 6 liegen.

Der innen liegende Zylinder 31 weist an seinem stromabwärts gelegenen Ende 35 einen Kragen auf, welcher eine Breite von r"' aufweist. Diese Breite ist verantwortlich für das Volumen des Hohlraums 36, welcher zwischen dem innen liegenden Zylinder und dem außen liegenden Zylinder ausgebildet wird. In diesem Hohlraum befindet sich der Rotor 13, welcher als einzelner Permanentmagnetring 37 mit vier Segmenten ausgebildet ist und die Segmente alternierend diametral nach außen oder diametral nach innen gerichtet polarisiert sind. Der Permanentmagnetring 37 trägt zur axialen Lagerung des Laufrads 9 auf dem Leitkörper 8 bei, indem die axiale Komponente des sich zwischen dem Rotor 13 und dem Stator ausbildenden Magnetfelds einem axialen Versatz aufgrund des herrschenden Flüssigkeitsdruckunterschieds in der Tangentialkammer und dem axialen Einlass entgegenwirkt.

Bei der Herstellung des Laufrads 9 wird (bzw. werden falls vorhanden zunächst die Permanentmagneten 15 und) der Rotor 13 auf den innen liegenden Zylinder 31 aufgeschoben und dieser Verbund mit dem außen liegenden Zylinder 32 verbunden und anschließend mit einem ringartigen Deckel 38 verschlossen. Anschließend wird das Laufrad 9 (oder eine andere Variante des Laufrads) in das Rohr 2 eingeführt oder der Leitkörper in die Öffnung des Laufrads geschoben.

Die Lage des Rotors 13 ist im vorliegenden Beispiel derart gewählt, dass der Rotor 13 im Wesentlichen dem Stator 13 des Leitkörpers 8 axial direkt gegenüberliegt. Hierdurch kann die Kraftkopplung zwischen dem Rotor 13 und dem Stator 12 den bestmöglichen Wirkungsgrad entfalten.

Im vorliegenden Beispiel bedeutet dies, dass der Rotor 13 derart in dem Laufrad angeordnet ist, dass dieser beim angetriebenen Laufrad 9 vollständig innerhalb des Hohlraums 4 des Rohres 2 liegt. Selbstverständlich können jedoch auch Teile des Rotors derart im Hohlraum 36 des Laufrads 9 angeordnet sein, dass dieser zumindest teilweise in die Kavität 5 der Spiralkammer 3 reicht.

In der Fig. 5b ist ein Querschnitt des Rotors 13 gezeigt, wobei insbesondere ein segmentierter, aus einzeln magnetisierten Segmenten gebildeter Permanentmagnetring 37 sichtbar ist. Der Permanentmagnetring weist alternierend verschieden magnetisierte Segmente 39, 40, 41 und 42 auf, wobei die Segmente 39 und 41 radial oder diametral nach innen und die Abschnitte 40 und 42 radial oder diametral nach außen polarisiert sind. Mit dem hier dargestellten Permanentring 37 ist ein Vierpolbetrieb des Motors möglich. Die einzelnen Abschnitte 39 bis 42 haben jeweils eine effektive Remanenz von mehr als 0,5 T. Um die Kopplung zu verbessern können allgemein Segmente mit Remanenzen von mehr als 0.5 T, 1 T, 1.2 T oder 1.4 T eingesetzt werden.

Anhand der Fign. 6a und 6b soll auf eine weitere Pumpenvariante und allgemein die Lagerung des Laufrads 9 auf dem Leitkörper 8 näher eingegangen werden.

Es ist deutlich zu erkennen, dass in der Pumpenvariante der Fign. 6 keine zusätzlichen axialen Lagerelemente vorgesehen sind. Die axiale Lagerung wird in dieser Variante durch die Wechselwirkung zwischen Stator und Rotor bewirkt, wobei die axiale Komponente des koppelnden Magnetfelds einer das Laufrad vom Dorn drückenden Kraft entgegenwirkt. Diese Kraft resultiert im Wesentlichen aus der passiven axialen Zentrierwirkung des Permanentmagnetrings relativ zum Statorjoch. Diese Kraft kann durch geeignete Maßnahmen, z.B. Schrägstellung der Nutung des Stators bzw. durch schräge Polarisierung des Rotors, vergrößert werden. Außer den fehlenden axialen Lagermagneten ist der Aufbau der Pumpe der Fign. 6 im Wesentlichen mit dem Pumpenaufbau der Fig. 1 vergleichbar.

Eine Außenfläche 43 des Leitkörpers 8 ist durch einen Spalt mit einer radialen Breite von einigen Hundert Mikrometern von der Innenfläche 34 des Laufrads 9 getrennt. Sowohl die Oberfläche 43 als auch die Oberfläche 34 können hierbei Ausnehmungen aufweisen, so dass zwischen den beiden Oberflächen ein hydrodynamisches Lager ausgebildet wird, wenn das Laufrad mithilfe des Motors in Rotation versetzt wird. Weitere, beispielsweise magnetische Radiallager sind nicht vorgesehen, ebenso wenig wie weitere Antriebs- oder Lagerkomponenten, welche radial außerhalb des Laufrads liegen.

Anhand der Fig. 5a ist ersichtlich, dass im Falle eines zylindermantelförmigen Leitkörpers 8 und im Falle eines zylinderförmigen Laufrads 9 beide Elemente auch gänzlich in einer Spiralkammer vorgehalten werden können. Hierzu kann beispielsweise alternativ eine den Radialpumpen ähnliche Beschaufelung vorgesehen werden. Der Vorteil gegenüber handelsüblichen Modellen liegt darin, dass aufgrund der zueinander parallel verlaufenden Oberflächen die Kraftkopplung zwischen dem Laufrad bzw. dem Rotor des Laufrads und dem Stator des Leitkörpers maximiert wird und die Lagerung des Laufrads besonders einfach ist. Hierdurch können die Kosten zur Herstellung einer Blutpumpe wesentlich reduziert werden. Des Weiteren ist es auch möglich, beispielsweise ein zusätzliches radiales Magnetlager im Leitkörper 8 vorzuhalten. Dies kann dabei das in der Fig. 5a als reines hydrodynamisches Lager ausgebildete Lager zwischen den Oberflächen 34 und 43 vollständig oder teilweise ersetzen.

In der Fig. 6a ist deutlich erkennbar, dass der Stator 12 im Teilbereich 8.2 des Leitkörpers 8 angeordnet ist, wobei der Teilbereich 8.2 sich zu mehr als 90% außerhalb der Spiralkammer 3 befindet. Im Zusammenspiel mit dem Rohr 2 ergibt sich somit nicht nur der Spalt zwischen den Oberflächen 34 und 43 sondern auch ein Spalt zwischen dem radialen Rand der Beschaufelung 20 des Laufrads 9 und der Innenwand des Rohes 2. Hierdurch kann bei entsprechender Abmessung ein Dämpfungsglied bereitgestellt werden. In der vorliegenden Variante trägt der Spalt jedoch nicht nennenswert zur hydrodynamischen Lagerung bei.

Eine weitere Variante eines rein hydrodynamischen Lagers kann der Fig. 6b entnommen werden. Der Leitkörper 45 weist in seinem mittleren Abschnitt 46 einen kleineren Durchmesser gegenüber den randseitigen Bereichen 47 und 48 auf. Allen drei Bereichen 46 bis 48 ist gemein, dass diese koaxial zur Achse 6 ausgebildet sind. Eine durch das Laufrad 9 überdeckte Oberfläche 49 des Abschnitts 48 des Leitkörpers 45 bildet mit dem gegenüberliegenden Teil 50 der Oberfläche 34 einen ersten Abschnitt eines hydrodynamischen Radiallagers. Analog hierzu bildet sich zwischen der Oberfläche 51 des Abschnitts 47 und der dieser Oberfläche gegenüberliegenden Teiloberfläche 52 ein weiterer Abschnitt des hydrodynamischen Radiallagers aus. Auch der mittlere Abschnitt 46 und die diesem gegenüberliegende Teiloberfläche 53 können ein Teil des hydrodynamischen Radiallagers sein. Vorliegend sind die Teiloberfläche 53 und der mittlere Abschnitt 46 derart voneinander beabstandet, dass sie nicht (bzw. nicht nennenswert) zur hydrodynamischen Lagers beitragen, da ein ununterbrochenes Radiallager im Pumpenbetrieb zwar steifer, jedoch energetisch ungünstiger ist. Das hydrodynamische Radiallager umfasst mehrere einander benachbarte Zylinderflächen, welche den stabilen Lauf des Laufrads auf dem Leitkörper 45 sicherstellen.

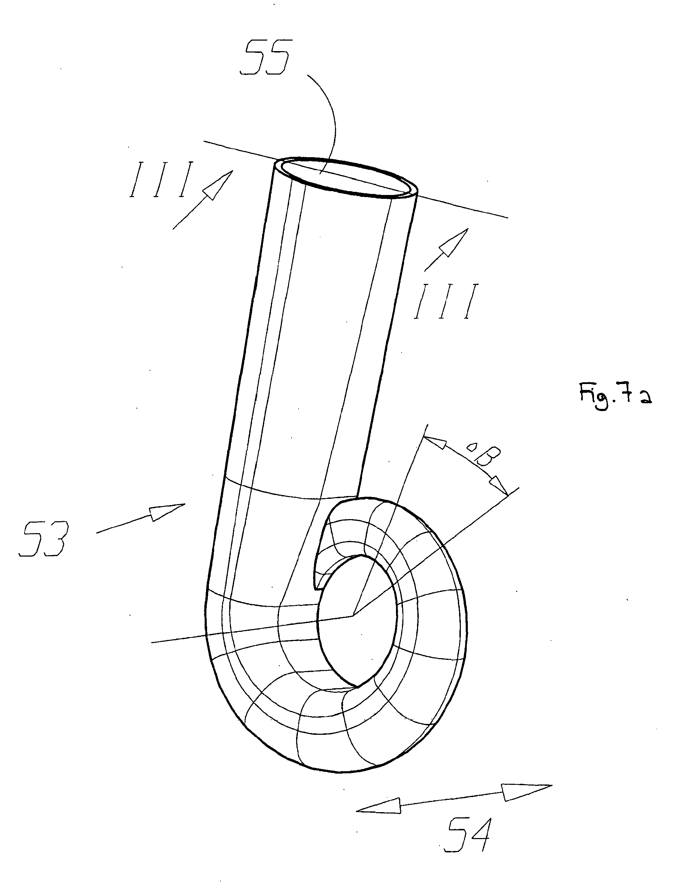

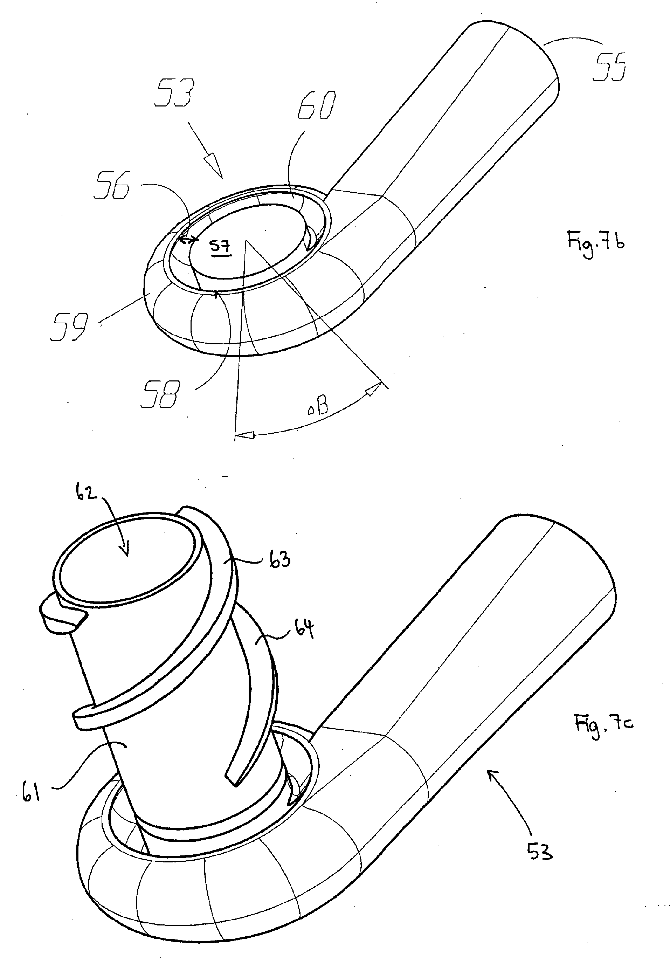

In den Fign. 7 wird exemplarisch eine Schneckenkammer dargestellt. Die Schneckenkammer 53 ist in der Fig. 7a von der Rückseite und in der Fig. 7b von der Vorderseite her entlang des Schnitts III der Schneckenkammer der Fig. 7a von der Vorderseite her dargestellt.

In der Fig. 7a ist deutlich zu erkennen, dass die axiale Ausdehnung 54 der Kavität bzw. Kammer der Schneckenkammer 53 pro Winkelelement Δβ im Uhrzeigersinn laufend zum radialen Auslass 55 hin zunimmt. Wie anhand der Fig. 7b erkennbar ist, nimmt die radiale Ausdehnung 56 zwischen der soliden Platte 57 und dem die Schneckenkammer 53 nach außen begrenzenden Rand 58 nicht zu, sondern bleibt konstant. Lediglich die axiale Ausdehnung der Kavität 59 verändert sich vom der Zunge 60 in der Fig. 7b gegen den Uhrzeigersinn laufend pro Winkelelement Δβ. Mit der Schneckenkammer 53 ist eine besonders schonende Blutförderung möglich. Ein Zapfen zur Aufnahme des Laufrads wird auf der Platte 57 angeordnet. Dies ist in der Fig. 7c illustriert, bei welcher das Laufrad 61 auf einen Dorn bzw. Zapfen 62 aufgeschoben ist, wobei der Dorn 62 auf der Platte 57 montiert ist. Zudem ist erkennbar, dass die als Wendel ausgeführte Beschaufelung zwei Schaufeln 63, 64 mit sich in axialer Richtung verändernder Ganghöhe umfasst. Eine Kappe wie in der Fig. 1 ist nicht oder noch nicht auf den Dorn aufgesetzt.

In der Fig. 8 ist ein Querschnitt einer Variante einer Doppelspiralkammer dargestellt, welche als Tangentialkammer einer erfindungsgemäßen Pumpe, einer Pumpe mit außenliegendem Stator wie in der PCT/EP2010/006863 bzw. US 61/258,932 gezeigt oder als selbständige Erfindung verwendbar ist (dies gilt auch für eine nachstehend beschriebene N-fachkammer). Die Doppelspiralkammer 65 umfasst zwei Zungen 66, 67, welche zwei Strömungspfade 68 bzw. 69 bewirken. Die Strömungspfade münden am Ende 70 der Zunge 67 ineinander und streben aus dem Auslass. Durch die einem axialen Einlass zugewandte, eingezeichnete Öffnung 71 erstreckt sich im montierten Zustand ein Laufrad mit zwei Schaufeln, welche auf dem Laufrad jeweils um 180° zueinander versetzt enden. Ein beispielhaftes Laufrad ist in der Fig. 7c dargestellt. Korrespondierend zur Beschaufelung des Laufrads sind die Zungen 66 bzw. 67 derart angeordnet, dass diese um 180° zueinander versetzt sind. Beim Überstreichen einer Zunge 66 durch eine Schaufel 63 wird gleichzeitig die gegenüberliegende Zunge 67 durch die korrespondierende Schaufel 64 überstrichen. Auf diese Weise entstehen die zwei Strömungspfade 68 bzw. 69. Mittels einer Doppelspiralkammer ist eine besonders schonende Blutförderung und Laufruhe gegeben. Analog zu der hier dargestellten Doppelspiralkammer können ebenfalls Dreifach- oder N-fachspiralkammer verwendet werden, deren Zungen vorzugsweise einen Versatz von etwa 120° bzw. etwa 360°/N zueinander aufweisen und die verschiedenen Strömungspfade zumindest zum Auslass hin zusammenführen. Vorzugsweise weist das Laufrad dann eine korrespondierende Anzahl von Schaufeln auf.

An dieser Stelle sei darauf hingewiesen, dass, obgleich in den vorliegenden Ausführungsbeispielen lediglich zylinderförmige Leitkörper und zylinderförmige Laufräder dargestellt wurden, prinzipiell auch kegelstumpfmantelförmige Laufräder und kegelstumpfförmige Leitkörper mit Hohlräumen möglich sind. Hierbei ist jedoch zu beachten, dass die Lagerung des Laufrads auf dem Leitkörper durch ein hydrodynamisches Lager gebildet wird, welches gegebenenfalls auch durch die radiale Berandung der Beschaufelung und der Innenwand eines Rohres, in welches sowohl der Leitkörper als auch das Laufrad ragen, vorhanden ist.

## Patentansprüche

1. Blutpumpe (1), umfassend
einen axialen rohrförmigen Einlass (2),
eine an den axialen Einlass (2) anschließende Tangentialkammer (3) mit im Wesentlichen radial gerichtetem Auslass (18),
einen mit dem Einlass (2) und/oder der Tangentialkammer (3) verbundenen axialen Leitkörper (8; 45),
ein auf dem Leitkörper (8; 45) gelagertes Laufrad (9) mit einer dem Leitkörper zugewandten Innenfläche (34) und einer Außenfläche,
eine auf der Außenfläche (33) angeordneten Beschaufelung (20)
sowie einen Motor, welcher einen dem Leitkörper (8; 45) zugeordneten Stator (12) und einen dem Laufrad (9) zugeordneten Rotor (13) umfasst,
**dadurch gekennzeichnet,**
**dass** zumindest ein Teilbereich (8.1; 8.2) des Leitkörpers von der Tangentialkammer (3) in den Einlass (2) ragt und zumindest ein Kraft auf den Rotor (13) übertragender Abschnitt des Stators (12) in dem Teilbereich (8.1; 8.2) angeordnet ist und das Laufrad (9) in radialer Richtung durch ein hydrodynamisches Radiallager gelagert ist.

2. Blutpumpe nach dem vorhergehenden Anspruch, **dadurch gekennzeichnet, dass** das Laufrad (9) in radialer Richtung ausschließlich durch ein vorzugsweise zwischen der Innenfläche (34) des Laufrads und der Leitkörperaußenfläche (43; 49, 51) gebildetes hydrodynamisches Radiallager gelagert ist.

3. Blutpumpe nach einem der vorhergehenden Ansprüche, **dadurch gekennzeichnet, dass** der Stator (12) in einem Hohlraum (11) des Leitkörpers (8; 45) angeordnet ist.

4. Blutpumpe nach einem der vorhergehenden Ansprüche, **dadurch gekennzeichnet, dass** der Leitkörper (8; 45) mit einer Wand (7) der Tangentialkammer (3) verbunden ist.

5. Blutpumpe nach einem der vorhergehenden Ansprüche, **dadurch gekennzeichnet, dass** der Stator (12) Wicklungen (23, 24) und vorzugsweise einen Eisenkern (25, 26) umfasst.

6. Blutpumpe nach einem der vorhergehenden Ansprüche, **dadurch gekennzeichnet, dass** das Laufrad (9) im Teilabschnitt (8.1; 8.2), vorzugsweise vollständig, zylindermantelförmig ausgebildet ist.

7. Blutpumpe nach einem der vorhergehenden Ansprüche, **dadurch gekennzeichnet, dass** das Laufrad (9) einen Hohlraum (36) umfasst und der Rotor (13) im Hohlraum (36) angeordnet ist.

8. Blutpumpe nach einem der vorhergehenden Ansprüche, **dadurch gekennzeichnet, dass** der Rotor (13) vorzugsweise mindestens einen alternierend magnetisierten segmentierten Permanentmagnetring (37) umfasst.

9. Blutpumpe nach Anspruch 8, **dadurch gekennzeichnet, dass** der Permanentmagnetring als einstückiger Ringmagnet vorliegt oder aus jeweils einzeln magnetisierten Teilsegmenten zusammengesetzt ist.

10. Blutpumpe nach einem der vorhergehenden Ansprüche, **dadurch gekennzeichnet, dass** das Laufrad (9) derart angeordnet ist, dass die Beschaufelung (20) sowohl in der Tangentialkammer (3) als auch im Einlass (2) angeordnet ist.

11. Blutpumpe nach Anspruch 10, **dadurch gekennzeichnet, dass** die Beschaufelung (20) eine auf der Außenfläche (33) angeordnete Wendel umfasst.

12. Blutpumpe nach einem der vorhergehenden Ansprüche, **dadurch gekennzeichnet, dass** die Innenfläche (34) des Laufrads (9) eine zylinderförmige Innenfläche ist und/oder die Außenfläche (33) des Laufrads (9) eine zylinderförmige Lauffläche ist.

13. Blutpumpe nach einem der vorhergehenden Ansprüche, **dadurch gekennzeichnet, dass** das hydrodynamische Radiallager durch Ausnehmungen auf der Leitkörperaußenfläche (43; 49; 51) und/oder der Innenfläche (34) des Laufrads (9) gebildet ist.

14. Blutpumpe nach einem der vorhergehenden Ansprüche, **dadurch gekennzeichnet, dass** das hydrodynamische Radiallager zwei oder mehr Paare von Innenflächenabschnitten (50, 52) des Laufrads (9) und jeweils dazu korrespondierenden Leitkörperaußenflächenabschnitten (49; 51) umfasst.

15. Blutpumpe nach einem der vorhergehenden Ansprüche, **dadurch gekennzeichnet, dass** das Laufrad (9) und der Leitkörper (8; 45) derart ausgebildet sind, dass das Laufrad (9) in einer axialen Richtung durch ein vorzugsweise passives magnetisches Axiallager gelagert ist.

16. Blutpumpe nach dem Anspruch 15, **dadurch gekennzeichnet, dass** das Laufrad (9) und der Leitkörper (8; 45) derart ausgebildet sind, dass das Laufrad (9) in einer axialen Richtung durch den entsprechend ausgelegten Motor und/oder das Laufrad (9) in einer axialen Richtung durch nicht dem Motor zugehörige Permanentmagnetsysteme (14, 15) axial gelagert ist.

## Claims

1. A blood pump (1) comprising
an axial tubular inlet (2),
a tangential chamber (3) adjoining the axial inlet (2) and having a substantially radially directed outlet (18),
an axial guide body (8; 45) connected to the inlet (2) and/or to the tangential chamber (3), an impeller (9) supported on the guide body (8; 45) and having an inner surface (34) facing the guide body and an outer surface,
blading (20) arranged on the outer surface (33), and a motor which includes a stator (12) associated with the guide body (8; 45) and a rotor (13) associated with the impeller (9),
**characterized in that**
at least one part region (8.1; 8.2) of the guide body projects from the tangential chamber (3) into the inlet (2) and at least one section of the stator (12) transmitting force onto the rotor (13) is arranged in the part region (8.1; 8.2) and the impeller (9) is supported by a hydrodynamic radial bearing in the radial direction.

2. A blood pump in accordance with the preceding claim, **characterized in that** the impeller (9) is supported in the radial direction only by a hydrodynamic radial bearing preferably formed between the inner surface (34) of the impeller and the guide body outer surface (43; 49, 51).

3. A blood pump in accordance with one of the preceding claims, **characterized in that** the stator (12) is arranged in a hollow space (11) of the guide body (8 ; 45).

4. A blood pump in accordance with one of the preceding claims, **characterized in that** the guide body (8; 45) is connected to a wall (7) of the tangential chamber (3).

5. A blood pump in accordance with one of the preceding claims, **characterized in that** the stator (12) includes windings (23, 24) and preferably a ferrite core (25, 26).

6. A blood pump in accordance with one of the preceding claims, **characterized in that** the impeller (9) is made in cylinder jacket form in the part section (8.1; 8.2), preferably completely.

7. A blood pump in accordance with one of the preceding claims, **characterized in that** the impeller (9) includes a hollow space (36) and the rotor (13) is arranged in the hollow space (36) .

8. A blood pump in accordance with one of the preceding claims, **characterized in that** the rotor (13) preferably includes at least one alternatingly magnetized segmented permanent magnet ring (37).

9. A blood pump in accordance with claim 8, **characterized in that** the permanent magnet ring is present as a one-piece ring magnet or is composed of respective individually magnetized part segments.

10. A blood pump in accordance with one of the preceding claims, **characterized in that** the impeller (9) is arranged such that the blading (20) is arranged both in the tangential chamber (3) and in the inlet (2).

11. A blood pump in accordance with claim 10, **characterized in that** the blading (20) includes a helix arranged on the outer surface (33).

12. A blood pump in accordance with one of the preceding claims, **characterized in that** the inner surface (34) of the impeller (9) is a cylindrical inner surface and/or **in that** the outer surface (33) of the impeller (9) is a cylindrical running surface.

13. A blood pump in accordance with one of the preceding claims, **characterized in that** the hydrodynamic radial bearing is formed by cutouts on the guide body outer surface (43; 49; 51) and/or on the inner surface (34) of the impeller (9).

14. A blood pump in accordance with one of the preceding claims, **characterized in that** the hydrodynamic radial bearing includes two or more pairs of inner surface sections (50, 52) of the impeller (9) and respective guide body outer surface sections (49; 51) corresponding thereto.

15. A blood pump in accordance with one of the preceding claims, **characterized in that** the impeller (9) and the guide body (8; 45) are designed such that the impeller (9) is supported by a preferably passive magnetic axial bearing in an axial direction.

16. A blood pump according to claim 15, **characterized in that** the impeller (9) and the guide body (8; 45) are designed such that the impeller (9) is axially supported in an axial direction by the correspondingly configured motor and/or the impeller (9) is axially supported by permanent magnet systems (14, 15) not belonging to the motor.

## Revendications

1. Pompe sanguine (1), comprenant
une entrée tubulaire axiale (2),
une chambre tangentielle (3) disposée à la suite de l'entrée axiale (2), avec une sortie (18) orientée essentiellement radialement,
un corps de guidage axial (8 ; 45) relié à l'entrée (2) et/ou à la chambre tangentielle (3),
une roue à aubes (9) montée sur le corps de guidage (8 ; 45), avec une surface intérieure (34) tournée vers le corps de guidage et une surface extérieure ;
un aubage (20) monté sur la surface extérieure (33), ainsi qu'un moteur comprenant un stator (12) attribué au corps de guidage (8 ; 45) et un rotor (13) attribué à la roue à aubes (9),
**caractérisée en ce que**
au moins une région partielle (8.1 ; 8.2) du corps de guidage fait saillie dans l'entrée (2) à partir de la chambre tangentielle (3), et **en ce qu'**au moins une section du stator (12) transmettant une force au rotor (13) est agencée dans la région partielle (8.1 ; 8.2), et **en ce que** la roue à aubes (9) est soutenue dans la direction radiale par un palier radial hydrodynamique.

2. Pompe sanguine selon l'une des revendications précédentes, **caractérisée en ce que** dans la direction radiale, la roue à aubes (9) est soutenue exclusivement par palier radial hydrodynamique formé de préférence entre la surface intérieure (34) de la roue à aubes et la surface extérieure du corps de guidage (43 ; 49, 51).

3. Pompe sanguine selon l'une des revendications précédentes, **caractérisée en ce que** le stator (12) est installé dans un espace creux (11) du corps de guidage (8 ; 45).

4. Pompe sanguine selon l'une des revendications précédentes, **caractérisée en ce que** le corps de guidage (8 ; 45) est relié à une paroi (7) de la chambre tangentielle (3).

5. Pompe sanguine selon l'une des revendications précédentes, **caractérisée en ce que** le stator (12) comprend des enroulements (23, 24) et de préférence un noyau de fer (25, 26).

6. Pompe sanguine selon l'une des revendications précédentes, **caractérisée en ce que** la roue à aubes (9) est formée comme une enveloppe de cylindre dans la région partielle (8.1 ; 8.2), de préférence entièrement.

7. Pompe sanguine selon l'une des revendications précédentes, **caractérisée en ce que** la roue à aubes (9) comprend un espace creux (36) et le rotor (13) est installé dans l'espace creux (36).

8. Pompe sanguine selon l'une des revendications précédentes, **caractérisée en ce que** le rotor (13) comprend de préférence au moins un anneau à aimant permanent (37) segmenté et aimanté en alternance.

9. Pompe sanguine selon la revendication 8, **caractérisée en ce que** l'anneau à aimant permanent se présente comme un aimant annulaire monopièce, ou se compose de segments partiels respectivement aimantés séparément.

10. Pompe sanguine selon l'une des revendications précédentes, **caractérisée en ce que** la roue à aubes (9) est montée de manière à ce que l'aubage (20) soit montée aussi bien dans la chambre tangentielle (3) que dans l'entrée (2).

11. Pompe sanguine selon la revendication 10, **caractérisée en ce que** l'aubage (20) comporte des spires agencés sur la surface extérieure (33).

12. Pompe sanguine selon l'une des revendications précédentes, **caractérisée en ce que** la surface intérieure (34) de la roue à aubes (9) est une surface intérieure cylindrique et/ou **en ce que** la surface extérieure (33) de la roue à aubes (9) est une surface de roulement cylindrique.

13. Pompe sanguine selon l'une des revendications précédentes, **caractérisée en ce que** le palier radial hydrodynamique est formé par des évidements dans la surface extérieure du corps de guidage (43 ; 49, 51) et/ou la surface intérieure (34) de la roue à aubes (9) .

14. Pompe sanguine selon l'une des revendications précédentes, **caractérisée en ce que** le palier radial hydrodynamique comprend deux paires ou plus de sections de surface intérieure (50, 52) de la roue à aubes (9) et des sections de surface extérieure de corps de guidage (49 ; 51) respectivement correspondantes.

15. Pompe sanguine selon l'une des revendications précédentes, **caractérisée en ce que** la roue à aubes (9) et le corps de guidage (8 ; 45) sont conçus de manière à ce que la roue à aubes (9) soit soutenue dans une direction axiale par un palier axial de préférence aimanté passif.

16. Pompe sanguine selon la revendication 15, **caractérisée en ce que** la roue à aubes (9) et le corps de guidage (8 ; 45) sont conçus de manière à ce que la roue à aubes (9) soit soutenue axialement par le moteur conçu en conséquence et/ou à ce que la roue à aubes (9) soit soutenue dans une direction axiale par des systèmes à aimant permanent (14, 15) non-attribués au moteur.
